# EUROPEAN PATENT SPECIFICATION

(11) **EP 4 027 875 B1**
(45) Date of publication and mention of the grant of the patent: **03.01.2024**
(21) Application number: 20893243.4
(22) Date of filing: 25.11.2020
(51) Int. Cl.: A61B 5/05, A61B 5/06, A61B 6/00, A61B 6/03, A61B 90/00, A61B 1/04

(54) **SENSOR-LESS DC MOTOR CLOSED LOOP CONTROLLER FOR IMAGING CAPSULE**
SENSORLOSE GLEICHSTROMMOTORSTEUERUNG MIT GESCHLOSSENEM REGELKREIS FÜR EINE BILDGEBUNGSKAPSEL
DISPOSITIF DE COMMANDE À BOUCLE FERMÉE DE MOTEUR CC SANS CAPTEUR POUR CAPSULE D'IMAGERIE

(30) Priority: 25.11.2019 US 201962939709 P
(43) Date of publication of application: 20.07.2022
(73) Proprietor: Check-Cap Ltd., 30090 Isfiya (IL)
(72) Inventor: KIMCHY, Yoav, 3475715 Haifa (IL); HAZUT, Ofer, 6215004 Tel Aviv (IL); CHEN, Elik, 22350 Naharia (IL)
(74) Representative: Forresters IP LLP
(86) International application number: PCT/IL2020/051213
(87) International publication number: WO 2021/105987

(56) References cited:
- WO-A1-2018/140683
- US-A- 4 595 014
- US-A1- 2006 052 708
- US-A1- 2010 174 184
- US-A1- 2014 037 069
- US-A1- 2014 037 069
- US-A1- 2019 343 425
- US-B1- 8 068 897

## Description

### RELATED APPLICATIONS

The present application claims priority from US Provisional application number 62/939,709 filed on November 25, 2019.

### FIELD OF THE INVENTION

The present invention relates generally to rotation of a radiation scanner in an imaging capsule and more specifically to rotating with a brushed DC motor.

### BACKGROUND OF THE INVENTION

One method for examining the gastrointestinal tract for the existence of polyps and other clinically relevant features that may provide an indication regarding the potential of cancer is performed by swallowing an imaging capsule that will travel through the tract and view the patient's situation. In a typical case the trip can take between 24-48 hours after, which the imaging capsule exits in the patient's feces. Typically the patient swallows a contrast agent to enhance the imaging ability of the imaging capsule. Then the patient swallows the imaging capsule to examine the gastrointestinal tract while flowing through the contrast agent. The imaging capsule typically includes a radiation source, for example including a radioisotope that emits X-rays or Gamma rays. Likewise the imaging capsule includes a detector to detect particles from X-ray fluorescence and/or Compton back-scattering that are reflected responsive to the radiation emitted from the radiation source.

The radiation is typically collimated to allow it to be controllably directed toward a specific area during the imaging process. Typically the imaging capsule includes a motor to rotate the collimator and controllably scan the inner wall of the colon or small bowel.

The imaging capsule records the measurements and transmits measurements (e.g. a count rate) to an external analysis device, for example a computer or other dedicated instruments for analysis and reconstruction of an image of the wall of the colon and/or small bowel.

US Patent application No. 7,787,926 to Kimchy describes details related to the manufacture and use of such an imaging capsule.

It is desirable that the motor will be as simple and small as possible to minimize the size of the capsule. On the other hand the imaging capsule needs to be able to accurately determine the direction of the collimator and detector to be able to reconstruct an accurate image of the wall of the colon and/or small bowel.

Patent application publication US 2010/0174184 dated July 8, 2010 describes an apparatus and method for detecting clinically relevant features in the gastrointestinal tract.

Patent application publication US 2014/0037069 dated February 6, 2014 describes fail-safe radiation concealment mechanisms for imaging capsules.

Patent application US 4,595,014 dated June 17, 1986 describes an apparatus and method for imaging a radiation pattern from within a body.

### SUMMARY OF THE INVENTION

An aspect of an embodiment of the invention, relates to an imaging capsule for scanning with radiation within a living body using a brushed DC motor. The motor comprises a segmented commutator and is configured to provide a pulse signal that indicates motion of the brushes from segment to segment. The pulsed signal thus provides an accurate indication of an angle of rotation of the motor. Based on the pulsed signal the imaging capsule can determine a count rate for each angular sector and thus provide an accurate scan of an inner wall of a user's colon.

There is thus provided according to an embodiment of the disclosure, an imaging capsule, comprising:
A radiation source;
A collimator that provides a collimated beam from the radiation source;
A detector configured to detect particles resulting from X-ray fluorescence and/or Compton backscattering in response to the collimated beam;
A motor configured to rotate the collimator and detector around an axle to scan a partial or full inner circumference of a user's colon with radiation;
Wherein the motor comprises a segmented commutator that is fed with a power signal via brush contacts; and
Wherein the motor provides a pulsed output signal based on mechanical switching of the segmented commutator on the brush contacts, providing an indication of the rotation angle of the motor as a function of time.

In an embodiment of the disclosure, the imaging capsule reconstructs an image by determining a photon count per unit of angular sector. Optionally, the power signal is a pulse width modulation (PWM) signal. In an embodiment of the disclosure, the motor feeds one or more gears to increase momentum and reduce rotation speed of the collimator. Optionally, the imaging capsule includes a shutter to selectively block emission of radiation from the collimator; and wherein the shutter is opened by powering the motor and closed by powering the motor with reversed polarity. In an embodiment of the disclosure, the shutter is closed if the battery voltage falls below a preset threshold voltage. Optionally, the shutter is closed by a backup power source. In an embodiment of the disclosure, the pulsed output signal is processed by a high pass filter and a low pass filter. Optionally, the start and stop time for data acquisition from the radiation detectors is determined based on the pulsed output signal from the motor. In an embodiment of the disclosure, a sensor is used to confirm when the motor is in a specific angular position.

### BRIEF DESCRIPTION OF THE DRAWINGS

The present invention will be understood and better appreciated from the following detailed description taken in conjunction with the drawings. Identical structures, elements or parts, which appear in more than one figure, are generally labeled with the same or similar number in all the figures in which they appear, wherein:
Fig. 1A is a schematic illustration of a cross sectional view of an imaging capsule with a DC motor, according to an embodiment of the disclosure;
Fig. 1B is a schematic illustration of an internal perspective view of an imaging capsule with a DC motor, according to an embodiment of the disclosure;
Fig. 2 is a schematic illustration of an internal view of a motor, according to an embodiment of the disclosure;
Fig. 3 is a schematic illustration of an electronic circuit including a safety backup electronic circuit, according to an embodiment of the disclosure;
Fig. 4 is a schematic illustration of a circuit for providing power to motor and an output signal from motor, according to an embodiment of the disclosure; and
Fig. 5 is a trace of a pulse signal from the motor contact brushes in response to the power signal from the battery, according to an embodiment of the disclosure.

### DETAILED DESCRIPTION

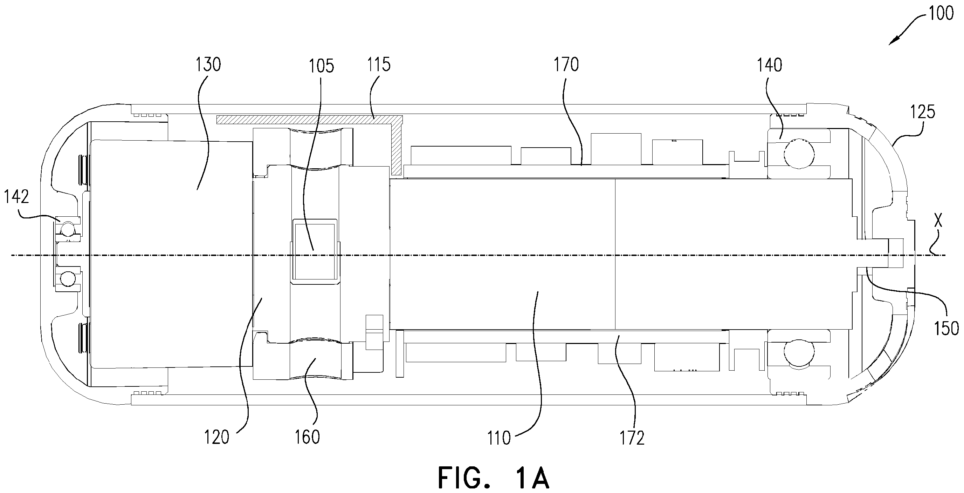

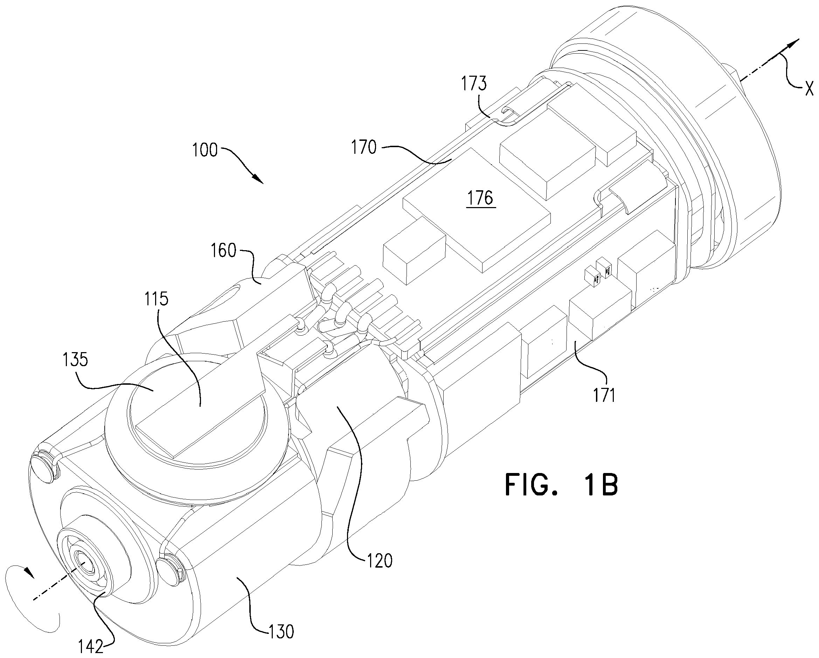

Fig. 1 is a schematic illustration of a cross sectional view of an imaging capsule 100 with a DC motor 110, and Fig. 1B is a schematic illustration of an internal perspective view of an imaging capsule 100 with a DC motor 110, according to an embodiment of the disclosure. In an embodiment of the disclosure, the imaging capsule 100 is configured to be swallowed by the user to examine the user's gastrointestinal tract in vivo. The imaging capsule 100 includes a radiation source 105 (e.g. emitting X-rays and/or Gamma rays) and a collimator 120 to control the direction of release of the radiation. Additionally, the imaging capsule 100 includes one or more detectors 115 to count particles resulting from X-ray fluorescence and/or Compton backscattering. Optionally, a shutter 160 may be coupled to the collimator 120 to selectively release or block radiation from being emitted from collimator 120.

In an embodiment of the disclosure, the imaging capsule is enclosed within an encasement 125. A motor 110 is used to rotate the internal elements of the imaging capsule 100 within the encasement 125. The rotated internal elements include the radiation source 105, collimator 120 and detectors 115 to scan an inner circumference of a wall of the user's colon, small intestine and/or other organs within the gastrointestinal tract. A signal from the motor 110 is used to accurately determine a rotation angle of the collimator 120 and detector 115 as a function of time. The rotation angle is used to identify what position (e.g. an angular sector) of the circumference is being scanned at any given moment. The photon count of the detector and rotation angle from the motor are used to reconstruct an image of the inner wall of the user's gastrointestinal tract (e.g. the inner wall of the colon).

In an embodiment of the disclosure, the motor 110 is mounted on a front bearing 140 and a rear bearing 142, which are configured to support motor 110 as it rotates around axle 150 that coincides with an elongated axis X. Optionally, the motor 110 is powered by a battery 130, which provides DC current or a PWM (pulse width modulation) signal and may also rotate with the motor 110.

In an embodiment of the disclosure, circuit boards (170, 171, 172, 173) are coupled to the motor, for example to regulate the input current to the motor 110, accept an output signal from the motor 110, analyze measurements and communicate with an external device. Optionally, imaging capsule 100 provides measurements to the external device and/or receives information and instructions from the external device, while traversing the gastrointestinal tract.

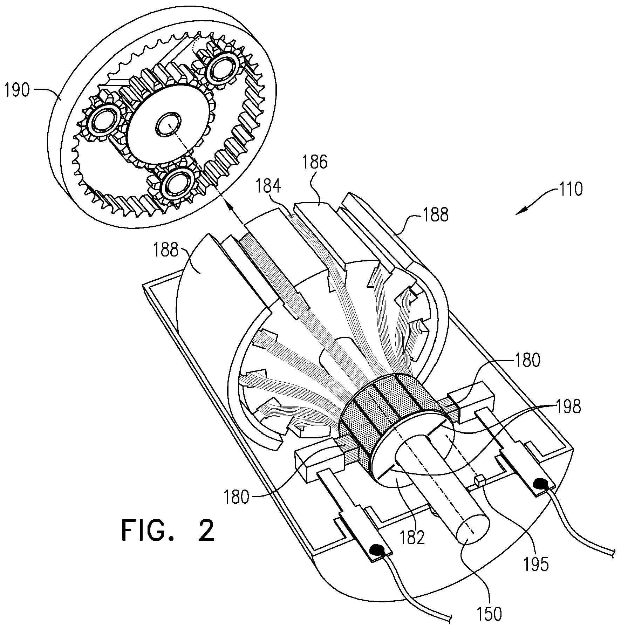

Fig. 2 is a schematic illustration of an internal view of a motor 110, according to an embodiment of the disclosure. In an embodiment of the disclosure, motor 110 is a DC motor with brush contacts 180 that provide current to a segmented commutator 182. The segmented commutator 182 provides current to an armature 184 to cause the rotor 186 to rotate relative to the stator 188. Optionally, the axle 150 of the motor 110 is coupled to a gear 190 (e.g. a planetary gear or other type of gear) to reduce the resulting rotation speed of the collimator due to rotations of the motor while increasing the torque to rotate the collimator 120 and the other internal elements of the imaging capsule 100. The gear enables consumption of a minimal amount of energy while providing sufficient angular momentum for rotating the internal elements of the imaging capsule 100. Optionally, the motor 110 may rotate many cycles (e.g. 10, 100, 1000) to cause the collimator to perform a single cycle. In an embodiment of the disclosure, the motor may be programmed to perform 100 cycles in a first direction to cause the collimator to perform 80% of a cycle and then swap the rotation direction to cause the collimator 120 to return to a starting position. Optionally, the collimator may release radiation to scan simultaneously in multiple directions so that the imaging capsule may perform more than one scan in a single forward/backward sweep of the collimator.

In an embodiment of the disclosure, a DC motor with brush contacts and a planetary gear with a high gear ratio (e.g. 1:136) such as TT Motor TGPP06-C-136 (from China) is used as motor 110 to rotate the internal elements of imaging capsule 100. In an embodiment of the disclosure, the shutter 160 opens and closes as a function of the rotation angle of the motor. Optionally, the high gear ratio ensures a high friction of the mechanical elements of the imaging capsule 100 so that when the shutter 160 is closed and no power is applied, the shutter 160 will remain shut and not accidently open and release radiation.

The use of a DC motor with brushes allows the imaging capsule 100 to close the shutter 160 by applying a DC voltage with reverse polarity to the motor 110. In some embodiments this is achieved by using a backup power source 135, for example in the form of a super capacitor such as Elna DSK-3R3H703T414-HRL. Optionally, backup power source 135 is connected to a separate backup electronic circuit (e.g. circuit 171) that is wired to the motor connection leads. Optionally, when receiving a fault signal or lack of signal (e.g. after waiting a preset amount of time with no response) from a main circuit (e.g. circuit 170) the backup power source 135 provides power to close shutter 160 and prevent emission of radiation.

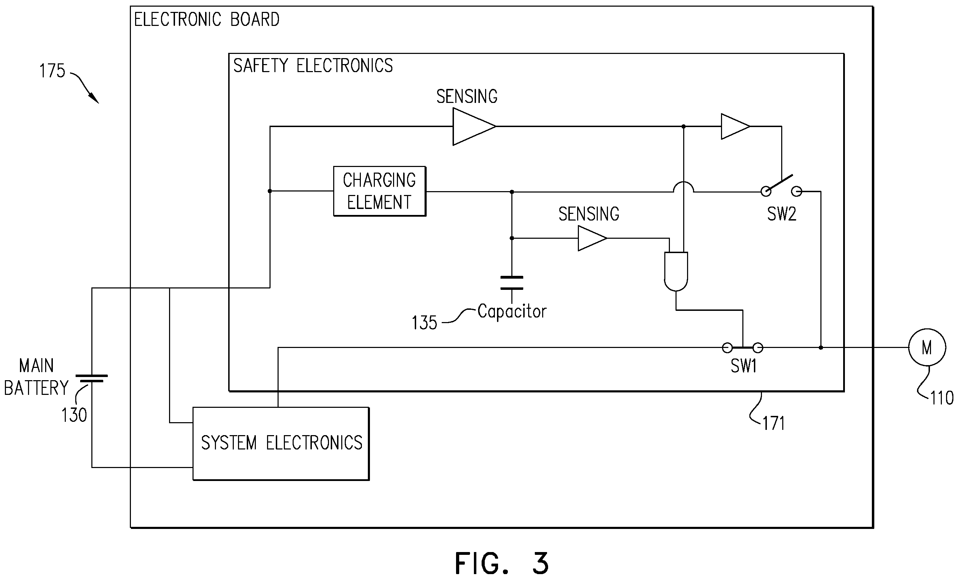

Alternatively, backup electronic circuit 171 can use power from battery 130 when a fault occurs or if the battery voltage that is continuously monitored by backup electronic circuit 171 falls below a preset threshold value. Optionally, backup circuit 171 then uses the remaining power to close shutter 160 and prevent further release of radiation. Fig. 3 is a schematic illustration of an electronic circuit 175 including a safety backup electronic circuit 171, according to an embodiment of the disclosure. In Fig. 3 if the power from the battery 130 fails, then switch SW1 opens and switch SW2 closes and power is provided to motor 110 from capacitor 135 instead of from the battery 130.

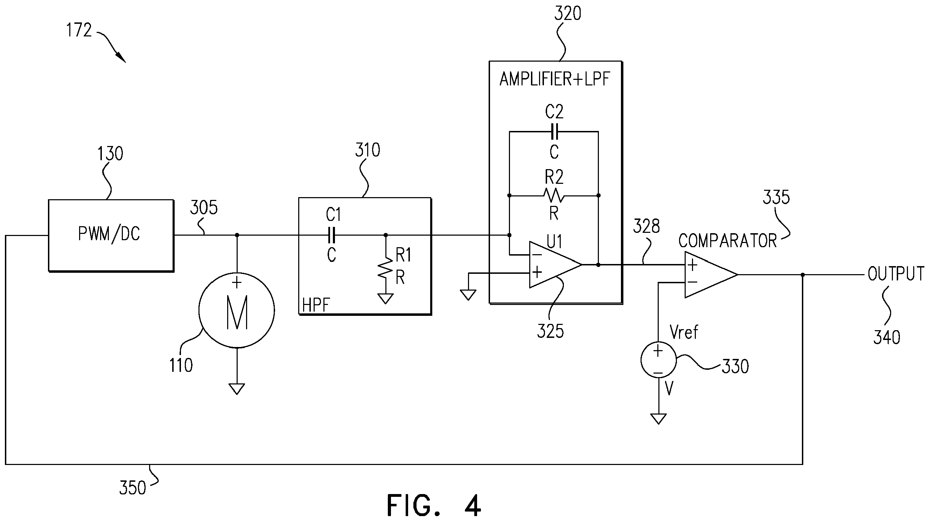

Fig. 4 is a schematic illustration of a circuit 172 for providing power to motor 110 and an output signal 340 from motor 110, according to an embodiment of the disclosure. Optionally, the output signal 340 from motor 110 is used to accurately determine the rotational angle of the motor signal. In an embodiment of the disclosure, circuit 172 receives power from battery 130 and provides a DC voltage, a DAC signal or a PWM signal to power motor 110. Optionally, the motor brush contacts 180 are connected to a high pass filter 310 and a low pass filter 320 with a sensing amplifier 325 to provide a truncated pulse signal at 328. The truncated signal is compared to a reference voltage 330 with a comparator 335 to verify that the motor is functioning as expected. The pulse signal at 328 demonstrates the sensing of the mechanical switching of the segments of the commutator 182 on the brushes 180. This configuration allows closed loop control of the motor angular speed based on the number of pulses per unit time that are detected from the motor brushes 180. Optionally, the speed is controlled by increasing or decreasing the voltage supplied to the motor based on a feedback line 350. In an embodiment of the disclosure, the pulsed signal at output 340 from circuit 172 is also used by the capsule electronics to control the start and stop time for data acquisition from the radiation detectors 115 in the capsule. Accordingly, the imaging capsule 100 can measure photons received per a unit of angular sector, since the detector 115 is in sync with the motor 110. This provides better homogeneity of the imaging scan relative to an imaging capsule where the imaging sector is determined by timing only and/or the motor runs in an open loop.

In an embodiment of the disclosure, a sensor 195 such as an optical sensor, magnetic sensor or a contact sensor is placed to accurately identify one or more positions along the rotational scan of the motor. Sensor 195 is used as an angular position index for the motor control electronics to mechanically confirm a specific angular position of the motor. The segmented commutator 182 may include markings 198 at specific angular positions to be identified by sensor 195. At the specific angular position, the control electronics sets the angular position counter to the preset angular position, so that drifts in angular position measurements of the pulses from the motor brushes 180 can be reduced and not accumulated over the scan time. In an embodiment of the disclosure, the motor 110 is configured to drive the collimator back and forth around axle 150 while the imaging capsule 100 is scanning the insides of the user's gastrointestinal tract, for example the user's colon. Optionally, the sensor 195 will be positioned to identify the beginning or end of the planned motion of the motor. In an embodiment of the disclosure, the software controlling the motor movement and counting the angular position based on the pulses coming from the brushes will set a position segment counter to zero each time the sensor reaches the beginning or end, and then start again from that known position.

In an embodiment of the disclosure, one of the circuits (e.g. circuit 170) may include a processor, memory and transceiver to serve as a controller 176 that is programmed to control image scanning performed by imaging capsule 100. Optionally, the controller 176 will continuously monitor the current drawn by the motor 110. The controller software will rotate the motor 110, and with it the collimator 120, shutter 160 and the circuits (e.g. circuits 170, 171, 172, 173) until the collimator reaches the end of a rotational sector and meets a mechanical limit. For example the shutter 160 may open for a single cycle of the collimator (optionally, multiple cycles of the motor 110 as explained above) and is automatically pushed closed near the end of the cycle or after being reversed and returning to the beginning.

In an embodiment of the disclosure, meeting the mechanical limit causes the motor 110 to stall and increases the current drawn by the motor 110. This rise in motor current, when it passes a preset threshold indicates to the controller 176 that the mechanical limit has been reached. Additionally, at this point, when the motor stalls, the pulse signals from the brushes 180 cease indicating that the motor stalled. At this point, the controller 176 will stop supplying current to the motor and will reset the position counter to the position of the mechanical limit reached.

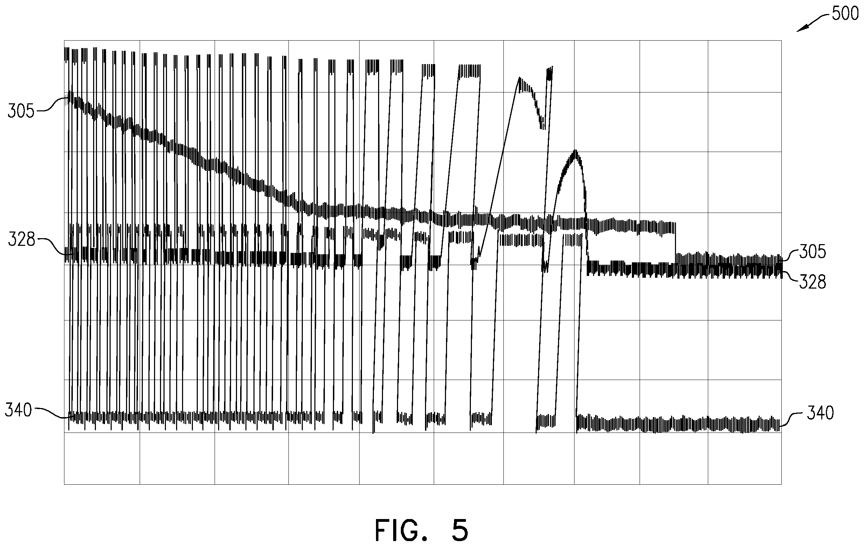

Fig. 5 is a trace 500 of the pulse signal 340 from the motor contact brushes 180 in response to the power signal 305 from the battery 130, according to an embodiment of the disclosure. In trace 500 the power signal 305 (e.g. a DC voltage signal in this case) is shown gradually declining before the motor stops. At the same time pulse signal 340 forms widening pulses due to the slowing down of the motor 110. Signal 328 shows the pulse signal 340 before it is straightened out by comparator 335. As can be seen the signal 328 becomes less defined as the motor 110 halts.

In another embodiment of the disclosure, the safety backup electronic circuit 171 that is responsible for the safety of the imaging capsule 100 will include a timeout mechanism that will check for a "live" signal from the motor controller. If the "live" signal is not detected until reaching a preset timeout interval, the backup electronic circuit 171 will disable the motor controller 176 and drive the motor 110 to the "collimator shut" position.

It should be appreciated that the above described methods and apparatus may be varied in many ways, including omitting or adding steps, changing the order of steps and the type of devices used. It should be appreciated that different features may be combined in different ways. In particular, not all the features shown above in a particular embodiment are necessary in every embodiment of the invention. Further combinations of the above features are also considered to be within the scope of some embodiments of the invention, provided that for the various possibilities indicated above, the resulting subject-matter falls within the scope of the invention as defined by the claims.

It will be appreciated by persons skilled in the art that the present invention is not limited to what has been particularly shown and described hereinabove. Rather the scope of the present invention is defined only by the claims, which follow.

## Claims

1. An imaging capsule (100), comprising:
a radiation source (105);
a collimator (120) configured to provide a collimated beam from the radiation source (105);
a detector (115) configured to detect particles resulting from X-ray fluorescence and/or Compton backscattering in response to the collimated beam;
a motor (110) configured to rotate the collimator (120) and detector (115) around an axle (150) to scan a partial or full inner circumference of a user's colon with radiation;
**characterized by** the motor (110) comprising a segmented commutator (182) configured to be fed with a power signal (305) via brush contacts (180); and
wherein the motor (110) is configured to provide a pulsed output signal (340) based on mechanical switching of the segmented commutator (182) on the brush contacts (180), providing an indication of the rotation angle of the motor (110) as a function of time.

2. The imaging capsule (100) of claim 1, wherein the imaging capsule (100) is configured to reconstruct an image by determining a photon count per unit of angular sector.

3. The imaging capsule (100) of claim 1, wherein the power signal (305) is a pulse width modulation (PWM) signal.

4. The imaging capsule (100) of claim 1, wherein the capsule (100) further comprises one or more gears (190) and the motor (110) is configured to feed the one or more gears to increase momentum and reduce rotation speed of the collimator (120).

5. The imaging capsule (100) of claim 1, wherein the imaging capsule (100) includes a shutter (160) for selectively blocking emission of radiation from the collimator (120); and wherein the imaging capsule further comprises means for opening the shutter (160) by powering the motor (110) and for closing the shutter (160) by powering the motor (110) with reversed polarity, and optionally wherein the capsule further comprises a battery, and means for closing the shutter (160) if the battery (130) voltage falls below a preset threshold voltage, or optionally wherein the imaging capsule further comprises a backup power source and means for closing the shutter (160) by the backup power source (135).

6. The imaging capsule (100) of claim 1, wherein the imaging capsule further comprises means for processing the pulsed output signal (340) by a high pass filter (310) and a low pass filter (320).

7. The imaging capsule (100) of claim 1, wherein the imaging capsule further comprises electronics configured to determine the start and stop time for data acquisition from the radiation detectors (115) based on the pulsed output signal (340) from the motor (110).

8. The imaging capsule (100) of claim 1, wherein the imaging capsule further comprises a sensor (195) and means for using the sensor to confirm when the motor (110) is in a specific angular position.

## Patentansprüche

1. Abbildungskapsel (100), umfassend:
eine Strahlungsquelle (105);
einen Kollimator (120), der so konfiguriert ist, dass er einen kollimierten Strahl von der Strahlungsquelle (105) bereitstellt;
einen Detektor (115), der so konfiguriert ist, dass er als Reaktion auf den kollimierten Strahl Partikel erkennt, die aus Röntgenfluoreszenz und/oder Compton-Rückstreuung resultieren;
einen Motor (110), der so konfiguriert ist, dass er den Kollimator (120) und den Detektor (115) um eine Achse (150) rotiert, um einen Teil- oder Gesamtinnenumfang des Grimmdarms eines Benutzers mit Strahlung abzutasten;
**dadurch gekennzeichnet, dass** der Motor (110) einen segmentierten Kommutator (182) umfasst, der über Bürstenkontakte (180) mit einem Leistungssignal (305) versorgt werden kann; und
wobei der Motor (110) so konfiguriert ist, dass er basierend auf mechanischem Schalten des segmentierten Kommutators (182) an die Bürstenkontakte (180) ein gepulstes Ausgangssignal (340) bereitstellt, das eine Anzeige des Drehwinkels des Motors (110) als Funktion der Zeit bereitstellt.

2. Abbildungskapsel (100) nach Anspruch 1, wobei die Abbildungskapsel (100) so konfiguriert ist, dass ein Bild durch Bestimmen einer Anzahl von Photonen pro Winkelsektoreinheit rekonstruiert wird.

3. Abbildungskapsel (100) nach Anspruch 1, wobei das Leistungssignal (305) ein Pulsweitenmodulationssignal (PWM) ist.

4. Abbildungskapsel (100) nach Anspruch 1, wobei die Kapsel (100) weiterhin ein oder mehrere Zahnräder (190) umfasst und der Motor (110) so konfiguriert ist, dass er die eine oder mehreren Zahnrädergetriebe antreibt, um die kinetische Energie zu erhöhen und die Rotationsgeschwindigkeit des Kollimators (120) zu reduzieren.

5. Abbildungskapsel (100) nach Anspruch 1, wobei die Abbildungskapsel (100) eine Blende (160) zum selektiven Blockieren der Emission von Strahlung aus dem Kollimator (120) umfasst; und wobei die Abbildungskapsel weiterhin Mittel zum Öffnen der Blende (160) durch Antreiben des Motors (110) und zum Schließen der Blende (160) durch Antreiben des Motors (110) mit umgekehrter Polarität umfasst, und wobei optional die Kapsel außerdem eine Batterie und Mittel zum Schließen der Blende (160) umfasst, wenn die Spannung der Batterie (130) unter eine vordefinierte Schwellenspannung fällt, oder wobei optional die Abbildungskapsel außerdem eine Notstromquelle und Mittel zum Schließen der Blende (160) durch die Notstromquelle (135) umfasst.

6. Abbildungskapsel (100) nach Anspruch 1, wobei die Abbildungskapsel weiterhin Mittel zum Verarbeiten des gepulsten Ausgangssignals (340) unter Verwendung eines Hochpassfilters (310) und eines Tiefpassfilters (320) umfasst.

7. Abbildungskapsel (100) nach Anspruch 1, wobei die Abbildungskapsel außerdem eine Elektronik zum Bestimmen der Startzeit und der Stoppzeit der Erfassung von Daten aus den Strahlungsdetektoren (115) auf der Grundlage des gepulsten Ausgangssignals (340) aus dem Motor (110) umfasst.

8. Abbildungskapsel (100) nach Anspruch 1, wobei die Abbildungskapsel außerdem einen Sensor (195) und Mittel zur Verwendung des Sensors zur Bestätigung, wenn sich der Motor (110) in einer spezifischen Winkelposition befindet, umfasst.

## Revendications

1. Capsule d'imagerie (100), comprenant :
une source de rayonnement (105) ;
un collimateur (120) conçu pour fournir un faisceau collimaté provenant de la source de rayonnement (105) ;
un détecteur (115) conçu pour détecter des particules résultant d'une fluorescence de rayons X et/ou d'une rétrodiffusion Compton en réponse au faisceau collimaté ;
un moteur (110) conçu pour faire tourner le collimateur (120) et le détecteur (115) sur un axe (150) afin de balayer une circonférence intérieure partielle ou complète du côlon d'un utilisateur avec un rayonnement ;
**caractérisé en ce que** le moteur (110) comprend un collecteur segmenté (182) conçu pour être alimenté avec un signal de puissance (305) par le biais de contacts à balais (180) ; et
le moteur (110) étant conçu pour fournir un signal de sortie pulsé (340) sur la base d'une commutation mécanique du collecteur segmenté (182) sur les contacts à balais (180), ce qui fournit une indication de l'angle de rotation du moteur (110) en fonction du temps.

2. Capsule d'imagerie (100) selon la revendication 1, la capsule d'imagerie (100) étant conçue pour reconstruire une image en déterminant un nombre de photons par unité de secteur angulaire.

3. Capsule d'imagerie (100) selon la revendication 1, le signal de puissance (305) étant un signal de modulation de largeur d'impulsion (PWM).

4. Capsule d'imagerie (100) selon la revendication 1, la capsule (100) comprenant en outre un ou plusieurs engrenages (190) et le moteur (110) étant conçu pour alimenter les un ou plusieurs engrenages afin d'augmenter l'énergie cinétique et réduire la vitesse de rotation du collimateur (120).

5. Capsule d'imagerie (100) selon la revendication 1, la capsule d'imagerie (100) comprenant un obturateur (160) destiné à bloquer sélectivement l'émission de rayonnement provenant du collimateur (120) ; et la capsule d'imagerie comprenant en outre des moyens destinés à ouvrir l'obturateur (160) en alimentant le moteur (110) et fermer l'obturateur (160) en alimentant le moteur (110) avec une polarité inversée, et éventuellement la capsule comprend en outre une batterie, et des moyens destinés à fermer l'obturateur (160) si la tension de la batterie (130) tombe au-dessous d'une tension seuil prédéfinie, ou éventuellement la capsule d'imagerie comprenant en outre une source d'alimentation de secours et des moyens destinés à fermer l'obturateur (160) par la source d'alimentation de secours. (135).

6. Capsule d'imagerie (100) selon la revendication 1, la capsule d'imagerie comprenant en outre des moyens destinés à traiter le signal de sortie pulsé (340) à l'aide d'un filtre passe-haut (310) et d'un filtre passe-bas (320).

7. Capsule d'imagerie (100) selon la revendication 1, la capsule d'imagerie comprenant en outre une électronique conçue pour déterminer l'instant de départ et l'instant d'arrêt de l'acquisition de données à partir des détecteurs de rayonnement (115) sur la base du signal de sortie pulsé (340) du moteur (110).

8. Capsule d'imagerie (100) selon la revendication 1, la capsule d'imagerie comprenant en outre un capteur (195) et des moyens destinés à utiliser le capteur pour confirmer l'instant où le moteur (110) est dans une position angulaire spécifique.
